# FASCICULE DE BREVET EUROPEEN

(11) **EP 2 032 175 B1**
(45) Date de publication et mention de la délivrance du brevet: **30.03.2011**
(21) Numéro de dépôt: 07803914.6
(22) Date de dépôt: 21.06.2007
(51) Int. Cl.: A61L 2/04

(54) **STÉRILISATION DE PRODUIT COSMÉTIQUE**
STERILISIERUNG VON KOSMETIKA
COSMETIC PRODUCT STERILIZATION

(30) Priorité: 23.06.2006 FR 0652617
(43) Date de publication de la demande: 11.03.2009
(73) Titulaire: CL TECH (société par actions simplifiée), 30100 Ales (FR)
(72) Inventeur: LOPEZ, Didier, 30100 Ales (FR); LOPEZ, Christophe, 30100 Ales (FR)
(74) Mandataire: Rhein, Alain
(86) Numéro de dépôt international: PCT/FR2007/051489
(87) Numéro de publication internationale: WO 2007/148022

(56) Documents cités:
- FR-A1- 2 774 911
- GB-A- 1 056 681
- JP-A- 11 137 644
- US-A- 5 976 592
- US-A1- 2002 164 159

## Description

La présente invention concerne un procédé de stérilisation de produit cosmétique, dans lequel ledit produit est constitué par un fluide mis en circulation sous pression au sein d'un circuit de stérilisation, selon la revendication 1.

La présente invention entre dans le domaine de la cosmétologie, en particulier dans la fabrication de produits cosmétiques.

L'invention concerne plus particulièrement un procédé de stérilisation de produits cosmétiques et son dispositif de mise en oeuvre.

Afin d'améliorer la conservation d'un produit cosmétique, entrent dans sa composition des conservateurs ayant notamment pour rôle de conserver les propriétés dudit produit tout en le protégeant des contaminations. Les conservateurs sont répertoriés dans une liste identifiée par des organismes agréés. Un groupe de conservateur utilisé sont les parabens qui empêchent la croissance des champignons et des bactéries. Toutefois, ces composants présentent l'inconvénient d'être nocifs pour l'organisme et présente un risque pour la santé des utilisateurs.

Les fabricants tentent donc de substituer les parabens par d'autres composés pouvant servir de conservateur. Toutefois, les produits utilisés ne sont pas toujours présents dans les listes autorisées et n'apportent pas entière satisfaction.

Il a donc été imaginé de stériliser les produits cosmétiques au lieu d'incorporer des conservateurs. Plusieurs procédés et dispositifs de l'état de la technique, décrits par exemple dans les documents JP 60025907 et JP 10025235, consistent en une stérilisation par chauffage du produit cosmétique dans un intervalle de températures comprises entre 50 et 80 degré Celsius. Toutefois, les dispositifs n'apportent pas entière satisfaction sur la longévité du produit et sont complexes à mettre en oeuvre.

Il a donc été imaginé une stérilisation à très haute température, de l'ordre de 135° à 150°, toujours au travers de la mise en circulation d'un fluide au travers d'un circuit traversant des bains de chauffage et de refroidissement. Un tel dispositif est décrit dans le document US 2002/164159 en vue de la stérilisation de plusieurs types de produit, comme cosmétique mais dont l'application se destine tout particulièrement à l'a stérilisation du lait.

Ce dispositif présente des inconvénients en ce qu'il est destiné à la stérilisation d'un unique produit. En effet, le temps de stérilisation est ici adapté en augmentant ou diminuant la longueur du circuit. Une même installation ne peut donc être dédiée qu'à un unique produit, d'une viscosité donnée. De plus, le temps de stérilisation est très élevé, de l'ordre de 80 minutes, au travers d'une circulation au sein d'un échangeur tubulaire à effet Joule. Les températures élevées combinée à ce temps long obligent une vitesse rapide de circulation du produit, de l'ordre de 5 mètre par seconde.

D'autres solutions ont été envisagées, par exemple dans le document US 5 976 592, ayant recours à un champ électromagnétique afin d'irradier le produit et ainsi le chauffer puis de le refroidir dans un bain d'eau et de glycol à environ 15 degrés Celsius. Un tel dispositif n'apporte pas entière satisfaction, notamment en raison de la complexité et du coût de revient et d'utilisation. De plus, une même installation est dédiée à la stérilisation d'un même et unique produit d'une viscosité donnée.

L'invention a pour but de pallier les inconvénients de l'état de la technique en proposant un procédé de fabrication de produit cosmétique qui s'affranchit de l'ajout de conservateur dans la composition d'un produit cosmétique.

De plus, une même installation permet la stérilisation de produits d'une densité ou viscosité différentes. En adaptant la pression de circulation du produit, la présente invention contrôle son temps de stérilisation, soit le chauffage et le refroidissement.

Pour ce faire, l'invention consiste à stériliser les produits cosmétiques par le passage rapide à haute température suivi d'un refroidissement immédiat au travers d'un dispositif spécifique.

L'invention concerne donc un procédé de stérilisation de produit cosmétique, selon la revendication 1.

L'invention concerne aussi le dispositif de mise en oeuvre d'un tel procédé de stérilisation, selon la revendication 5.

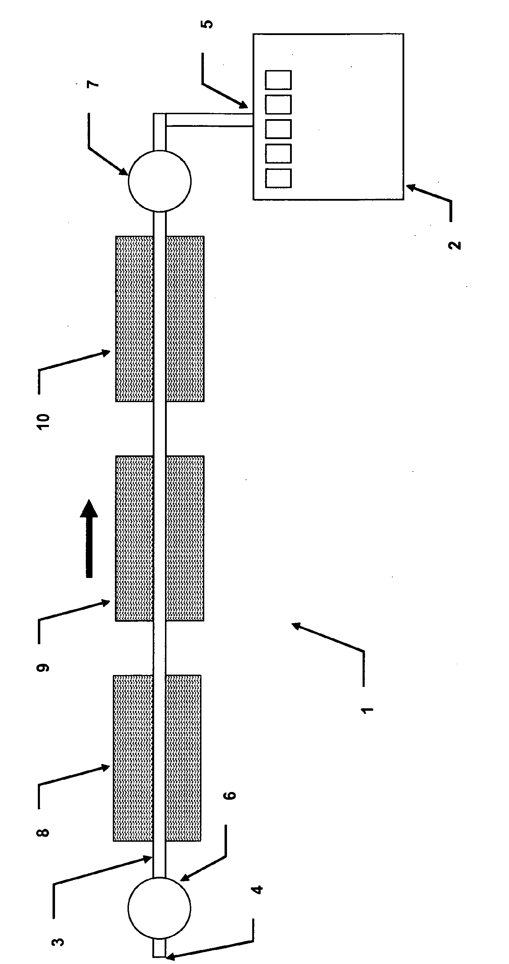

D'autres caractéristiques et avantages de l'invention ressortiront de la description détaillée qui va suivre des modes de réalisation non limitatifs de l'invention, en référence à la figure annexée représentant schématiquement les étapes de l'invention.

La présente invention concerne la stérilisation de produit cosmétique et réside dans la stérilisation à haute température dudit produit cosmétique suivi de son refroidissement immédiat.

Le principe stérilisation de l'invention se rapproche de la stérilisation de produit dans le domaine agro-alimentaire dit « UHT » pour ultra haute température.

La stérilisation UHT consiste à préchauffer un produit puis à le porter à des températures élevées pendant un court laps de temps avant de le refroidir à température ambiante, le produit ainsi traité étant alors emballé de façon aseptique.

La présente invention consiste donc à préchauffer le produit cosmétique, le chauffer brièvement à une température élevée puis à le refroidir.

Une caractéristique de l'invention réside dans le fait que le produit cosmétique est mis en circulation au sein d'un circuit de stérilisation 1. Le produit n'est dès lors plus en contact avec l'extérieur au cours de sa stérilisation et de son emballage, évitant tout risque de contamination après stérilisation.

En effet, le conditionnement du produit est réalisé sous une atmosphère stérile. Cette zone aseptique 2 de conditionnement peut préférentiellement comprendre un plafond soufflant de l'air filtré. Le conditionnement du produit y est réalisé sous vide, sans entrée d'air pour éviter tout contaminant.

Le circuit de stérilisation 1 est conçu apte à faire circuler sous pression un fluide. Pour ce faire, ledit circuit 1 se présente sous la forme d'une gaine 3 à l'intérieur de laquelle est mis sous pression le fluide depuis une entrée 4 vers une sortie 5.

En entrée 4, à une extrémité de ladite gaine, se trouve l'approvisionnement en produit et une pompe 6 permettant la mise sous pression dudit circuit 1.

En sortie 5, à l'extrémité opposée de ladite gaine, au niveau des moyens de conditionnement aseptique 2, se trouvent des moyens de régulation de la pression 7, notamment une vanne de régulation.

Selon un mode de réalisation, le circuit de stérilisation 1 comprend un serpentin à l'intérieur duquel circule le fluide de produit cosmétique. Ce serpentin peut préférentiellement avoir un diamètre intérieur de 5 millimètres.

Préférentiellement, selon un mode particulier de réalisation, ledit circuit 1 est constitué en matière inoxydable au travers d'une gaine de 5 à 10 mm de diamètre.

Les longueurs de serpentin dans chacun des bains, énoncés ultérieurement, sont identiques. De plus, la forme spécifique, notamment une forme hélicoïdale, assure une homogénéisation de chauffe et de refroidissement au coeur du produit y circulant.

On notera qu'une caractéristique essentielle de la présente invention réside donc dans l'obligation du produit cosmétique de se présenter sous forme d'un fluide. En fonction de sa viscosité, la pression à l'intérieur du circuit 1 est régulée. Selon le mode de réalisation et le fluide inséré dans le circuit 1, cette pression peut varier de 8 à 160 bars, de préférence entre 80 et 120 bars. Cette haute pression assure une meilleure émulsion des produits, en particulier des produits cosmétiques.

Le circuit de stérilisation 1 traverse successivement, mais non limitativement, des moyens de préchauffage 8, des moyens de chauffage 9 et des moyens de refroidissement 10.

Selon le mode préférentiel de réalisation, ces moyens de préchauffage 8, de chauffage 9 et de refroidissement 10 se présentent sous la forme de bain de produit porté à température, la température du produit dépendant du type de bain, chauffant ou refroidissant.

Selon le mode préférentiel de réalisation de l'invention, les moyens de refroidissement 10 comprennent un bain glycolé à -10 degrés Celsius, les moyens de préchauffage 8 comprennent un bain à 90 degrés Celsius afin d'assurer un chauffage au coeur du produit à au moins 70°c, tandis que les moyens de chauffage 9 comprennent un bain chaud à 180 degrés Celsius afin d'assurer un chauffage au coeur du produit à au moins 135°c. Toutefois, les valeurs des températures des différents bains peuvent varier singulièrement en fonction du produit à stériliser.

Dans la plupart des cas, la température des bains est maintenue stable et c'est uniquement la durée de passage du fluide à l'intérieur desdits bains qui varie. En effet, l'invention consiste à utiliser le déplacement du produit d'une zone thermique à une autre en régulant leur temps d'exposition.

Le passage dans le bain à très haute température est réalisé de manière rapide, sur une durée n'excédant pas quelques secondes. En particulier, le temps de passage dans les moyens de chauffage 9 peut être de 3 secondes.

Le temps de passage peut être régulé par variation de la pression, toujours en fonction de la viscosité du fluide traversant le circuit 1.

Le passage successif depuis une température élevée à une température très basse, de préférence négative, améliore la stérilisation et la conservation du produit. En effet, même si le produit ne passe pas par lui-même en température négative, ce refroidissement brutal évite la dégradation dudit produit.

En fin de compte, au travers d'un système de stérilisation similaire au procédé dit « UHT », la présente invention peut traiter en continue, ou sans arrêt, des produits cosmétiques de viscosités différentes sans modification de l'installation.

Le procédé de stérilisation selon l'invention trouvera une application particulière dans le cadre de gamme de produits cosmétiques à base d'eau thermale. En effet, la stérilisation à haute température permet de garder les minéraux et oligo-éléments présents dans l'eau. Dans cette gamme se trouvent notamment les produits suivants : les lotions aqueuses et nettoyantes, les gels nettoyants, les shampoings, les émulsions d'huile dans l'eau, etc.

## Revendications

1. Procédé de stérilisation de produit cosmétique, dans lequel ledit produit cosmétique est constitué par un fluide mis en circulation sous pression régulée, en fonction de la viscosité dudit fluide, au sein d'un circuit de stérilisation (1) sous forme d'un serpentin traversant des bains de produit porté à température, les longueurs de serpentin dans chacun desdits bains étant identiques consistant à successivement :
préchauffer ledit produit dans un bain de préchauffage ;
chauffer rapidement, de l'ordre de quelques secondes,
ledit produit dans un bain à haute température ;
puis refroidir immédiatement et brutalement ledit produit dans un bain froid de glycol ; et
conditionner le produit au sein d'une atmosphère stérile (2).

2. Procédé selon la revendication 1, **caractérisé par le fait que** le bain glycolé est à -10 degrés Celsius.

3. Procédé selon l'une quelconque des revendications 1, et 2, **caractérisé par le fait que** le bain des moyens de préchauffage (8) est à 70 degrés Celsius.

4. Procédé selon l'une quelconque des revendications 1 à 3, **caractérisé par le fait que** le bain chaud des moyens de chauffage (9) est à 135 degrés Celsius.

5. Dispositif de mise en oeuvre du procédé de stérilisation selon l'une quelconque des revendications 1 à 4 comprenant un circuit de stérilisation (1) apte à faire circuler sous pression régulée un fluide successivement au travers et comprenant :
des moyens (8) de préchauffage dudit fluide sous forme d'un bain de produit porté à température ;
des moyens (9) de chauffage à haute température dudit fluide sous forme d'un bain de produit porte à haute température ;
des moyens de refroidissement (10) dudit fluide, sous forme d'un bain de glycol, vers des moyens de conditionnement sous atmosphère stérile (2),
ledit circuit de stérilisation comprenant un serpentin, les longueurs de serpentin dans chacun desdits bains étant identiques.

6. Dispositif selon la revendication 5 **caractérisé par le fait que** ledit circuit (1) est constitué d'une gaine (3) dont l'une des extrémités comprend des moyens (6) de mise sous pression d'un fluide tandis que l'autre extrémité comprend des moyens (7) de régularisation de la pression.

## Claims

1. Method for sterilizing a cosmetic product, wherein said product is formed by a fluid circulated under regulated pressure, according to the viscosity of said fluid, in a sterilization circuit (1) in the form of a coil passing through baths of product brought at temperature, the coil lengths in each one of said baths being identical, consisting in successively :
preheating said product in a preheating bath ;
quickly heating, during some seconds, said product in a bath at high temperature, then immediately and abruptly cooling said product in a cold glycol bath ; and
conditioning the product in a sterile atmosphere (2).

2. Process according to claim 1, wherein the glycolyzed bath is at -10 degrees Centigrade.

3. Process according to any of the claims 1 and 2, wherein the bath of the preheating means (8) is at 70 degrees Centigrade.

4. Device according to any of the claims 1 to 3, wherein the hot bath of the heating means (9) is at 135 degrees Centigrade.

5. Device for implementing the sterilization method according to any of claims 1 to 4, comprising a sterilization circuit (1) capable of circulating a fluid under regulated pressure successively through, and comprising :
means (8) for preheating said fluid in the form of a bath of product brought at temperature ;
means (9) for heating said fluid at high temperature in the form of a bath of product brought at high temperature ;
means for cooling (10) said fluid, in the form of a glycol bath, to means for conditioning in a sterile atmosphere (2),
said sterilization circuit including a coil, the coil lengths in each one of said baths being identical.

6. Device according to claim 5, wherein said circuit (1) is formed by a conduit (3) one end of which comprises means (6) for putting a fluid under pressure, while the other end comprises means (7) for regulating the pressure.

## Patentansprüche

1. Verfahren zum Sterilisieren von kosmetischen Produkten, bei welchem das besagte kosmetische Produkt durch ein Medium gebildet ist, das unter in Abhängigkeit von der Viskosität des besagten Mediums gesteuerten Druck inmitten von einem Sterilisierungskreis (1) in Form von einer Rohrschlange, die Produktbäder durchquert, die auf eine bestimmte Temperatur gebracht sind, in Umlauf gebracht ist, wobei die Längen der Rohrschlange in jedem von den besagten Bädern identisch sind, bestehend aus den aufeinander folgenden Verfahrenschritten:
das besagte Produkt wird in einem Vorwärmbad vorerhitzt;
das besagte Produkt wird schnell, während wenigen Sekunden in einem Hochtemperatur-Bad hocherhitzt;
das besagte Produkt wird anschließend unverzüglich und schlagartig in einem Glykolkaltbad abgekühlt; und
das Produkt wird unter einer sterilen Umgebung (2) verpackt.

2. Verfahren nach Anspruch 1, **dadurch gekennzeichnet, dass** das Glykolbad bei -10 Grad Celsius liegt.

3. Verfahren nach irgendeinem der Ansprüche 1 und 2, **dadurch gekennzeichnet, dass** das Warmbad der Vorwärmmittel (8) bei 70 Grad Celsius liegt.

4. Verfahren nach irgendeinem der Ansprüche 1 bis 3, **dadurch gekennzeichnet, dass** das Heizbad der Heizmittel (9) bei 135 Grad Celsius liegt.

5. Vorrichtung für die Anwendung des Verfahrens zum Sterilisieren nach irgendeinem der Ansprüche 1 bis 4, umfassend einen Sterilisationskreis (1), geeignet, um ein Medium unter gesteuertem Druck aufeinander folgend durch Folgendes umlaufen zu lassen, und umfassend:
Mittel (8) zur Vorerwärmung des besagten Mediums in Form von einem auf eine bestimmte Temperatur gebrachte Produktbad;
Mittel zum Hocherhitzen (9) des besagten Mediums bei einer hohen Temperatur in Form von einem auf eine bestimmte Temperatur gebrachte Produktbad;
Mittel zum Abkühlen (10) des besagten Mediums in Form von einem Glykolbad, in Richtung zu den Mitteln zum Verpacken in einer sterilen Umgebung (2),
wobei der besagte Sterilisationskreis eine Rohrschlange umfasst, wobei die Längen der Rohrschlange in jedem der besagten Bäder identisch sind.

6. Vorrichtung nach Anspruch 5, **dadurch gekennzeichnet, dass** der besagte Kreis (1) durch ein Hüllrohr (3) gebildet ist, dessen ein Ende Mittel (6) zur Druckbeaufschlagung eines Mediums, während das andere Ende Mittel (7) für die Regelung des Drucks umfasst.
